# EUROPEAN PATENT SPECIFICATION

(11) **EP 1 071 321 B1**
(45) Date of publication and mention of the grant of the patent: **10.12.2008**
(21) Application number: 99922106.2
(22) Date of filing: 19.04.1999
(51) Int. Cl.: A01K 67/027, C12N 5/06, C12N 5/10

(54) **SOURCE OF NUCLEI FOR NUCLEAR TRANSFER**
QUELLE FÜR ZELLKERNE ZUM KERN-TRANSFER
SOURCES DE NOYAUX POUR TRANSFERT NUCLEAIRE

(30) Priority: 20.04.1998 GB 9808325
(43) Date of publication of application: 31.01.2001
(73) Proprietor: Consorzio Incremento Zootecnico S.R.L., 26100 Cremona (IT)
(72) Inventor: GALLI, Cesare, I-26100 Cremona (IT); LAZZARI, Giovanna, I-26100 Cremona (IT)
(74) Representative: Wakerley, Helen Rachael
(86) International application number: PCT/EP1999/002624
(87) International publication number: WO 1999/053751

(56) References cited:
- WO-A-97/07668
- WO-A-97/07669
- WO-A-98/07841
- WO-A-98/30683
- SCHNIEKE A ET AL: "Human Factor IX transgenic sheep produced by transfer of nuclei from transfected fetal fibroblasts" SCIENCE, vol. 278, 19 December 1997 (1997-12-19), pages 2130-2133, XP002067036
- RITCHIE W A ET AL: "INTRACYTOPLASMIC NUCLEAR INJECTION AS AN ALTERNATIVE TO CELL FUSION FOR THE PRODUCTION OF BOVINE EMBRYOS BY NUCLEAR TRANSFER" JOURNAL OF REPRODUCTION AND FERTILITY. SUPPLEMENT, vol. 5, 1 January 1995 (1995-01-01), page 60 XP000607293
- DU PASQUIER L ET AL: "Transplantation of nuclei from lymphocytes of adult frogs into enucleated eggs: special focus on technical parameters" DIFFERENTIATION, vol. 8, no. 1, 1977, pages 9-19, XP002115398
- KATO Y ET AL: "Eight calves cloned from somatic cells of a single adult" SCIENCE, vol. 282, no. 5396, 11 December 1998 (1998-12-11), pages 2095-2098, XP002115305

## Description

This invention relates to the generation of animals genetically identical to an existing or existed animal. Further, during the process of regeneration some characteristic(s) can be changed by recombinant DNA technology to produce a transgenic animal by the addition or deletion of selected genes.

Known procedures for nuclear transfer involve the transfer of a nucleus taken from a pre-implantation stage embryo into an enucleated mature oocyte. Following activation of the oocyte, in a process that mimics sperm entry and signalling, an embryo develops and eventually an individual that is genetically (as far as DNA is concerned) identical to the donor embryo. The limited number of cells present in a mammalian pre-implantation embryo, however, allows the regeneration of a limited number of embryos. Pre-implantation embryo nuclei donors do not allow the use of recombinant DNA technology because of the limited number of cells available. Most importantly, though, the genetic value of the embryo, and thus of the animal that will be born, can only be estimated. This is of low economic value. For these reasons the potential of nucleus transfer technology has not been developed with commercial exploitation in mind; its major use is for scientific purposes.

A partial solution to the limited number of nuclei has been the use of a so called 'serial nucleus transfer' where the embryos obtained from the starting embryos are further subjected once, or more than once, to the same procedure therefore increasing the number of embryos regenerated (Stice & al., 1991, Theriogenology 35, 273).

The major limitations to the use of nucleus transfer procedure outlined above would be overcome if a renewable and / or unlimited source of nuclei to be used in the process could be made available. For many years people have attempted to establish cell lines from pre-implantation embryos (embryonic stem cell lines) but failed except for the mouse. Such work is reviewed in Galli et al. 1994, Zygote 2: 385-389. This type of cell would represent the ideal source of nuclei, however in the mouse they have never been used in nucleus transfer experiments.

Cultured inner cell mass cells or presumptive embryonic cell lines have been obtained and successfully used for nucleus transfer experiments to produce embryos: Moor, Sun & Galli, 1992, Animal Reprod. Sci. 28, 423-431; Stice & al. 1996, Biol. Reprod. 54, 100-110. Viable offspring have been produced in cattle and sheep: Sims & First, 1994, Proc. Natl. Acad. Sci. USA 91: 6143-6147; Campbell & al. 1996, Nature 380, 64-66; Wells & al. 1997, Biol. Reprod. 57,385-393. More recently, viable offspring has also been obtained with the use of nuclei from cultured fetal cells: Wilmut & at. 1997, Nature 385, 810-813; see The New York Times 21 January 1998 and Nature 392, 113, 1998. One lamb has been produced from a sample taken from a primary culture containing mainly mammary epithelial cells of an adult sheep: Wilmut & at. 1997, Nature 385, 810-813.

The advantages of using a renewable source of cell or a cell line in nucleus transfer procedure are:
- cells can be easily collected and cultivated or possibly stored in liquid nitrogen;
- an unlimited number of embryos could be produced over a long period;
- cells can readily be modified in vitro using recombinant DNA technology.

There has been discussion about using nuclei of somatic cells collected from adult animals. This will have particular application for livestock species where the value of an animal is determined by his progeny if a sire or by her production records if, for example, a dam. To regenerate a unique animal for production or genetic characteristics (transgenic) it is imperative to use nuclei from an animal which is an adult or one which has at least been born alive. That is not the case for the work using fetal or embryonic cells as a source of nuclei.

### Description of the invention

The present invention provides a method of reconstructing a non-human mammalian embryo, the method comprising reconstructing a first generation embryo by transferring a mononuclear cell from the blood or natural secretion of a mammal into the cytoplasm of an enucleated oocyte; and transferring a cell from the said first generation embryo into the cytoplasm of an enucleated oocyte to form a second generation embryo.

Preferred features are set out in the accompanying claims. The mononuclear cell may be a lymphocyte. The cell can be transferred intact, optionally with a broken cell membrane, or the nucleus may be extracted and used for transfer. Preferably, a lymphocyte is transferred with the cell membrane broken.

Preferably, this invention finds application in the reconstruction of embryos of mammals using donor cells and recipients from the same species, preferably to reconstruct ungulate species embryos. The mononuclear cell may be collected from an adult animal or an animal from a viable birth.

The invention still further provides a method of preparing a non-human mammal, the method comprising reconstructing a non-human mammalian embryo using a method described above; allowing the embryo so produced to develop to term; and, optionally, breeding from the animal so formed.

The present invention further provides a method of preparing embryonic stem cell lines, comprising reconstructing a non-human mammalian embryo using a method described above; and transferring the embryo to a culture system.

The present invention further provides a method of preparing embryonic stem cell lines, comprising reconstructing a non-human mammalian embryo using a method described above; isolating the inner cell mass of the embryo from the embryo; and transferring the inner cell mass to a culture system.

The culture system allows the embryo cells to attach, outgrow and produce a cell line with embryonic characteristics. The term "embryo" used herein includes morulas (8-16 cells), morulas (16-32 cells) and blastocysts (64 cells and above). The embryo has a reasonable (about 50% or more) chance of development to an established pregnancy.

The present invention utilises lymphocytes and their derivatives or precursors. The donor cell, whilst usually a terminally differentiated hematopoietic cell, could be at a partially differentiated stage. These are mononuclear cells of hematopoietic lineage, present in bone marrow, lymphoid organs and in peripheral blood. They are also found in the umbilical cord of the new-born. The intact cells, cells with their membranes broken prior to transfer or the isolated nuclei are used as a source of nuclei in conventional nucleus transfer procedures. Lymphocytes can be collected from circulating blood, bone marrow, cord blood, lymphoid organs or natural secretions including milk and ejaculated semen. The sample can be enriched and purified by means of density gradient centrifugation or other means of separation, including immunomagnetic separation, fluorescence activated cell sorting, column filtration and similar techniques.

In the context of this invention, references to "mononuclear cells" for donor cells should be interpreted as including references to lymphocytes, being lymphocytes at more than 95% of the mononuclear cells separated on a Hystopaque gradient. Lymphocytes have been characterised by immunocytochemestry and do not express cytokeratins as well as lamin A/C that are typical of differentiated cells: Galli & al. 1995, Proc. of the Italian Soc. of Vet. Sci. XLIX, 303-304; Rober, RA & al., 1990, J. Cell Sci. 95, 587-598. To this extent, the hematopoietic lineage shares some characteristics with embryonic cells that are also negative for cytokeratins and lamin A/C: Galli et al. 1994, Zygote 2: 385-389. This could explain in part the successful reprogramming of these nuclei into the cytoplasm of enucleated matured oocytes.

Freshly collected lymphocytes can be cultured in vitro and are karyotypically normal. This latter characteristic is a prerequisite for the normal development of any individual, but it is not guaranteed by other cell types that have to be cultured for a length of time and where a degree of aneuploidy always occurs. Lymphocytes can also be cultured in vitro for a time sufficient to use recombinant DNA technology to alter their genetic constitution: Bordignon & al., 1995, Science 270, 470-475.

In principle this invention is applicable to all non-human mammals, but it will be useful in particular for livestock species such as cattle, buffaloes, sheep, goat, pigs, horses, rabbit and other species of economic relevance. It can also be used to preserve genetic material or to generate animals of endangered, exotic or rare species.

After the reconstruction procedure whereby a lymphocyte or the nucleus of a lymphocyte is reprogrammed into the cytoplasm of an enucleated oocyte, there are several options for which this invention could be used. Lymphocytes can be easily cryobanked and therefore offer an economic way of storing germplasm of animals. When the embryo is reconstructed it can be used not for reproduction but to generate undifferentiated embryonic cell lines to be used in cell therapy of the individual that donated them thus overcoming the problem of rejection. If the embryos obtained are used for the generation of an animal this can be done directly by transferring the pre-implantation embryos to a final recipient that will carry the embryo to term, or the embryo can be subjected to serial nucleus transfer and therefore generate further embryos in a process that is more efficient and probably will increase the chances of reprogramming the cell nucleus because is exposed to the egg's cytoplasm more than once in a short period.

The steps involved in the cloning of an animal using this invention are summarised:

Step 1 - isolate the donor cell required from circulating blood or other tissue; enrichment for the fraction of cells that is more efficient in the procedure; optionally the cells can be genetically modified during a period of in vitro culture using recombinant DNA technology.

At this stage the cells can be cultured, cryopreserved following one of the established protocols for later use or used immediately for nucleus transfer.

Step 2 - maturation of the oocytes harvested from donor females at slaughter or from live donors and removal of the egg's metaphase plate to prepare the so called 'recipient cytoplast'.

Step 3 - transfer of the nucleus obtained in step 1 by direct microinjection of the cell or of the isolated nucleus directly in the cytoplasm of the enucleated oocyte or by other means such as cell fusion that can be achieved using intact donor cells with chemical, electrical or viral means. Microinjection is preferred and, preferably, the cell is transferred with the cell membrane broken. Established cell fusion methods include the use of fusion-promoting chemicals, such as polyethylene glycol; the use of a virus such as the Sendai virus; and electrical stimulation.

After introduction of the lymphocyte, the oocyte is activated to mimic sperm entry and start the developmental programme of the oocyte. The delay if microinjection is used to introduce the lymphocyte is typically 2-6 hours before activation. Cold shock as well as aging can activate the cytoplast. Activation may also be by inducing calcium oscillations in the embryo by chemical (ionophore) or physical (electric current) means, following which the embryo is exposed to kinases and protein synthesis inhibitors that facilitate the exit from the metaphase arrest that is maintained upon new protein synthesis. Typical chemical activation would be by 6-dimethylamino purine or cycloheximide. This exposure would be subsequent to the ionophore and the exposure is typically for several hours (e.g., 4-6 hours).

Step 4 - develop the reconstructed embryo to a stage where it can be subject to a serial cloning procedure by disgregating the embryos obtained in single cells and restarting from step 2. Various known systems of culturing embryos can be used successfully.

The steps involved in the preparation of a stem cell line using this invention are summarised:

Obtain a preimplantation stage (morula or blastocyst, preferably a blastocyst) embryo following steps 1-4 described in the previous example.

Step 5 - Remove the zona pellucida of the embryo. Optionally, the inner cell mass may be isolated from the embryo, for example by mechanical means or by immunosurgery. The intact embryo or the isolated inner cell mass is plated and cultured. Various known systems of culturing embryonic stem cells may be used. The culture takes place on a monolayer of fibroblasts and/or in defined media supplemented with the necessary growth factors (leukaemia inhibitor factor, stem cell factor and others), which are required to maintain the embryonic cell in an undifferentiated state.

Step 6 - Subculture using, for example, mechanical or enzyme dispersal of the embryonic cell outgrowths in new culture vessels to expand the number of cells until a stable cell line is obtained.

Step 7 - The cell line may be frozen for long term storage or the genetic constitution of the cells genetically modified using recombinant DNA technology.

Step 8 - Following genetic modification, the embryonic cell may be used in the cloning of a mammal by following steps 2 to 4.

Recloning procedures can also be carried out by developing the embryo of steps 1-4 to the fetus stage in vivo and sampling cells from the fetus for use in the preparation of further embryos.

### EXAMPLE

This is an example of the use of the invention for the cloning of a cattle but similarly it can be applied to other species.

### Step 1 - Cell isolation

A blood sample was taken from a cow of proven genetic value by venipuncture with heparinized vacutainer. The blood was diluted 1:1 with phosphate buffer saline (PBS) and 7 ml were layered on 3 ml of a density gradient (Hystopaque density 1083 g/cm3, Sigma) and centrifuged at 1500g for 15-30 minutes, the mononuclear cells stopping at the plasma Hystopaque interface. The 0.5 - 1 ml band of mononuclear cells (lymphocytes) was recovered, transferred into a new centrifuge tube, further diluted with PBS and centrifuged again to wash the cells. This step was repeated once and the cells were finally resuspended in an appropriate culture medium.

Lymphocytes were cryopreserved in medium supplemented with 10-20% serum and 10% DMSO (dimethyl sulfoxide) and packed for example in plastic straws (normally used to pack bovine semen), each containing convenient working aliquots of cells (0.5-2 million cells) required in each day the method of the invention was carried out.

### Step 2 - Preparation of cytoplasts

Oocytes at the second metaphase were used. These oocytes were collected from ovaries of slaughtered animals or by ultrasound guided transvaginal recovery from live donors. After collection immature oocytes were subjected to a 15-20 hour maturation period until they reached the second metaphase, following protocols described by Galli & Lazzari, Anim. Reprod. Sci. 42, 371-379, 1996. Oocytes at the end of the maturation period were denuded from the surrounding follicle cells and treated with a fluorescent dye (Hoechst 33342) that stains the chromosomes in the metaphase plate. With the aid of a micromanipulator under an inverted microscope using a micropipette, the first polar body, with a small volume of cytoplasm surrounding it, was removed and checked under fluorescent light for the presence of the metaphase plate. After enucleation, the cytoplasts obtained in this way were returned to culture.

### Step 3 - Embryo reconstruction

Lymphocytes prepared in step 1 and cytoplasts prepared in step 2 were transferred to a manipulation chamber under an inverted microscope and each cytoplast was injected with a small micropipette with one cell as described in Tesarik & Mendoza, Human Reproduction 11, 772-779; 1996. It was important to make sure that the cytoplast membrane was broken and the cell or its nucleus was effectively injected in the cytoplasm ready to undergo the reprogramming events necessary to support embryonic development. Failure to break the cytoplast membrane adequately could leave the lymphocyte deposited in a "pocket" of the oocyte membrane. After injection the cytoplasts were returned to culture for a period generally of 2-4 hours.

About 70-80% of the cytoplasts survived the injection procedure. At this stage the oocytes were activated by exposing sequentially the reconstructed embryos (cytoplasts) for 5-7 minutes to 5 µM of Ionomycin (Sigma) and then to 2.5 mM 6-DMAP (Dimethyl amino purine, Sigma) for 4-5 hours: Susko-Parrish & al. 1994, Dev. Biol. 166, 729-739. This mimics sperm entry and will start the developmental programme of the oocyte.

### Step 4 - Embryo development

Following activation, the reconstructed embryos were transferred to an in vitro culture system generally used to develop fertilised oocytes to blastocysts. Embryos were cultured in microdrops of SOF (synthetic oviductal fluid, Gardner & al. 1994; Biol. Reprod, 50, 390-400) in an atmosphere of 5% CO₂, 5%O₂ in nitrogen at 38.5 °C.

### Reference Example - Embryo development

A proportion of the embryos (5%) developed to the blastocyst stage and could therefore be transferred to synchronised recipients or frozen for subsequent transfer.

With such embryos a pregnancy rate of over 50% (58%) was achieved, the most advanced stage obtained was a pregnancy aborted at 195 days of gestation (a normal bull calf of about 10 kg). The results are shown in Table 1. Most of the pregnancies resulted in abortions between 60-120 days. Two pregnancies developed to 180 days from 31 transfers. The first is the 195 day mentioned above, the second an oversized bull calf of 19kg which had to be aborted.

### Example: Second Generation Cloning

In the first generation cloning only 5% of the reconstructed embryos developed to the blastocyst stage. By this, 16 cell stage by day 4 and compacted morula by day 6 are presumably those embryos in which the reprogramming of the introduced nucleus has occurred.

To increase the efficiency of the procedure the first generation products are subjected to a second generation cloning. This second generation cloning is more efficient because it uses blastomeres (16, 32, 64 cells stages, preferably 32 or over cells stage) and also gives the DNA a second chance for reprogramming because it is recycled back into the cytoplast.

Embryos obtained in the first generation cloning were exposed to calcium and magnesium free HBSS (Hanks balanced salt solution) for 2-4 hours to separate the embryo into single isolated blastomeres.

Cytoplasts were prepared as described in step 2 and first activated (as described in step 3) before the blastomere nucleus was transferred. In this case, the intact blastomere was transferred to the perivitelline space of the cytoplast and electrofused. The fusion rate was usually high (in excess of 80%). Reconstructed embryos at this stage were transferred to the culture system described above. The results are shown in Table 1. 19 such recloned embryos were transferred and 10 pregnancies were established, a pregnancy rate of over 50% (53%).

A successful birth has been achieved; a live and healthy calf originating from an embryo of the second generation cloning. The original lymphocyte was collected from a bull.

**TABLE 1**

| Embryo development following lymphocyte injection or recloning. | | | | | |
|---|---|---|---|---|---|
| | No. replicates | No. injected | No. survived | No. cleaved | No. developed |
| | | | % | % | % |
| Direct injection | 25 | 1923 | 1377 | 1059 | 71 |
| (development to blastocyst) | | | 71.61 | 76.91 | 5.16 |
| Direct injection | 7 | 540 | 371 | 296 | 36 |
| (development to morula 16-64 cells) | | | 38.70 | 79.78 | 9.70 |
| Recloned from | 7 | 462* | 412 | 321 | 66 |
| morula | | | 89.18 | 77.91 | 16.02 |
| (development to blastocyst) | | | | | |

| Pregnancies | | Totals | Cloning | Recloning | |
|---|---|---|---|---|---|
| No. transfers | | 50 | 31 | 19 | |
| No. pregnancies | | 28 | 18 | 10 | |
| Pregnancy rate (%) | | 56.00 | 58.06 | 52.63 | |
| Developed to 90 days | | 10 | 5 | 5 | |
| Developed to 180 days | | 3 | 2 | 1 | |
| Developed to term | | 1 | 0 | 1 | |

| | | | | | |
|---|---|---|---|---|---|
| * "No. injected" is "No. fused" because recloning from blastomeres requires cell fusion not direct injection | | | | | |

### EFFECT OF THE INVENTION

The present invention provides a source of donor cells for nuclear transfer techniques which gives advantages over known donors. The use of lymphocytes makes for very easy sample collection, which can be from adult animals of known characteristics. The supply of donor cells is not limited. The donor cells can be readily modified in vitro using recombinant DNA technology.

## Claims

1. A method of reconstructing a non-human mammalian embryo comprising: reconstructing a first generation embryo by transferring a mononuclear cell from the blood or a natural secretion of a mammal into the cytoplasm of an enucleated oocyte; and transferring a cell from the said first generation embryo into the cytoplasm of an enucleated oocyte to form a second generation embryo.

2. A method according to claim 1 in which the first generation embryo is allowed to develop to the blastocyst stage before disgregation to obtain the cells used to form the second generation embryo.

3. A method according to claim 1 in which the first generation embryo is allowed to develop to the fetus stage in vivo before cells are sampled for use to form the second generation embryo.

4. A method according to claim 1, 2 or 3, in which the mononuclear cell is a lymphocyte.

5. The method according to any preceding claim further comprising the step of breaking the cell membrane of the mononuclear cell before transfer of said mononuclear cell into the enucleated oocyte.

6. Method according to any preceding claim, in which the mammal is an ungulate species.

7. Method according to any preceding claim further comprising the step of genetically modifying the nucleus of the mononuclear cell.

8. A method of preparing a non-human mammal, the method comprising:
reconstructing a non-human mammalian embryo using a method according to any preceding claim;
allowing the embryo so produced to develop to term; and
optionally breeding from the mammal so formed.

9. A method of preparing embryonic stem cell lines, comprising reconstructing a non-human mammalian embryo using a method according to any of claims 1 to 7 and transferring the embryo to a culture system.

10. A method of preparing embryonic stem cell lines, comprising reconstructing a non-human mammalian embryo using a method according to any of claims 1 to 7; isolating the inner cell mass of the embryo from the embryo and transferring the inner cell mass to a culture system.

11. A method according to claim 9 or 10 further comprising the step of genetic modification of the stem cells.

## Patentansprüche

1. Verfahren zum Rekonstruieren eines nicht humanen Säugerembryos, umfassend:
Rekonstruieren eines Embryos der ersten Generation mittels Transferieren einer mononukleären Zelle aus dem Blut oder einem natürlichen Sekret eines Säugers in das Zytoplasma einer entkernten Eizelle; und Transferieren einer Zelle aus dem Embryo der ersten Generation in das Zytoplasma einer entkernten Eizelle zur Bildung eines Embryos der zweiten Generation.

2. Verfahren nach Anspruch 1, worin sich der Embryo der ersten Generation vor der Disgregation zum Erhalt der zur Bildung des Embryos der zweiten Generation verwendeten Zellen bis zum Blastozystenstadium entwickeln darf.

3. Verfahren nach Anspruch 1, worin sich der Embryo der ersten Generation bis zum Fötusstadium *in vivo* entwickeln darf, bevor Zellen zur Verwendung zur Bildung des Embryos der zweiten Generation gewonnen werden.

4. Verfahren nach Anspruch 1, 2 oder 3, worin die mononukleäre Zelle einen Lymphozyten darstellt.

5. Verfahren nach einem der vorangehenden Ansprüche, das ferner den Schritt des Aufsprengens der Zellmembran der mononukleären Zelle vor dem Transfer der mononukleären Zelle in die entkernte Eizelle umfasst.

6. Verfahren nach einem der vorangehenden Ansprüche, worin der Säuger eine Huftierspezies darstellt.

7. Verfahren nach einem der vorangehenden Ansprüche, das ferner den Schritt des genetischen Modifizierens des Nukleus der mononukleären Zelle umfasst.

8. Verfahren zum Herstellen eines nicht humanen Säugers, wobei das Verfahren Folgendes umfasst:
Rekonstruieren eines nicht humanen Säugerembryos mithilfe eines Verfahrens nach einem der vorangehenden Ansprüche;
Entwickeln lassen des so erzeugten Embryos bis zum Termin; und
optional Züchten von dem auf diese Weise entstandenen Säuger.

9. Verfahren zum Herstellen embryonaler Stammzelllinien, umfassend das Rekonstruieren eines nicht humanen Säugerembryos mithilfe eines Verfahrens nach einem der Ansprüche 1 bis 7 und Transferieren des Embryos an ein Kultursystem.

10. Verfahren zum Herstellen embryonaler Stammzelllinien, umfassend das Rekonstruieren eines nicht humanen Säugerembryos mithilfe eines Verfahrens nach einem der Ansprüche 1 bis 7; Isolieren der inneren Zellmasse des Embryos aus dem Embryo und Transferieren der inneren Zellmasse an ein Kultursystem.

11. Verfahren nach Anspruch 9 oder 10, das ferner den Schritt der genetischen Modifikation der Stammzellen umfasst.

## Revendications

1. Méthode de reconstruction d'un embryon mammifère non humain comprenant : la reconstruction d'un embryon de première génération en transférant une cellule mononucléaire du sang ou d'une sécrétion naturelle d'un mammifère dans le cytoplasme d'un ovocyte énucléé ; et en transférant une cellule dudit embryon de première génération dans le cytoplasme d'un ovocyte énucléé pour former un embryon de seconde génération.

2. Méthode selon la revendication 1, dans laquelle on laisse l'embryon de première génération se développer jusqu'au stade blastocyste avant la désagrégation afin d'obtenir les cellules utilisées pour former l'embryon de seconde génération.

3. Méthode selon la revendication 1, dans laquelle on laisse l'embryon de première génération se développer in vivo jusqu'au stade foetal avant que les cellules ne soient prélevées pour les utiliser en vue de former l'embryon de seconde génération.

4. Méthode selon la revendication 1, 2 ou 3, dans laquelle la cellule mononucléaire est un lymphocyte.

5. Méthode selon l'une quelconque des revendications précédentes, comprenant, en outre, l'étape qui consiste à déchirer la membrane cellulaire de la cellule mononucléaire avant le transfert de ladite cellule mononucléaire dans l'ovocyte énucléé.

6. Méthode selon l'une quelconque des revendications précédentes, dans laquelle le mammifère fait partie de l'espèce des ongulés.

7. Méthode selon l'une quelconque des revendications précédentes, comprenant, en outre, l'étape qui consiste à modifier le noyau de la cellule mononucléaire génétiquement.

8. Méthode de préparation d'un mammifère non humain, ladite méthode comprenant :
la reconstruction d'un embryon de mammifère non humain en utilisant une méthode selon l'une quelconque des revendications précédentes ;
laisser l'embryon produit de la sorte se développer à terme;
et
reproduire optionnellement avec le mammifère ainsi formé.

9. Méthode de préparation de lignées de cellules souches embryonnaires, comprenant la reconstruction d'un embryon de mammifère non humain en utilisant la méthode selon l'une quelconque des revendications 1 à 7 et le transfert de l'embryon dans un système de culture.

10. Méthode de préparation de lignées de cellules souches embryonnaires, comprenant la reconstruction d'un embryon mammifère non humain en utilisant une méthode selon l'une quelconque des revendications 1 à 7 ; l'isolement de la masse cellulaire interne de l'embryon issue de ce dernier et le transfert de la masse cellulaire interne dans un système de culture.

11. Méthode selon la revendication 9 ou 10, comprenant, en outre, l'étape de la modification génétique des cellules souches.
